# EUROPEAN PATENT SPECIFICATION

(11) **EP 2 870 243 B1**
(45) Date of publication and mention of the grant of the patent: **14.02.2018**
(21) Application number: 13765472.9
(22) Date of filing: 08.07.2013
(51) Int. Cl.: C12N 11/02, C12N 11/06, C12N 11/08, C12P 19/02, C12P 19/14

(54) **ENZYMES IMMOBILISED ON STYRENE-DIVINYL BENZENE POLYMER MATRICES AND THE USE THEREOF IN INDUSTRIAL PRODUCTIONS**
AUF STYROL-DIVINYL-BENZOL-POLYMERMATRIZEN IMMOBILISIERTE ENZYME UND IHRE VERWENDUNG BEI INDUSTRIELLEN PRODUKTIONEN
ENZYMES IMMOBILISÉES SUR DES MATRICES DE POLYMÈRE DE STYRÈNE-DIVINYLBENZÈNE ET L'UTILISATION DE CELLES-CI DANS DES PRODUCTIONS INDUSTRIELLES

(30) Priority: 06.07.2012 IT FI20120139
(43) Date of publication of application: 13.05.2015
(73) Proprietor: INALCO S.R.L., 20139 Milano (IT)
(72) Inventor: CHINI, Jacopo, I-50131 Firenze (IT); FEBBRUARI, Barbara, I-51034 Cantagrillo (IT); MATULLI, Marina, I-40045 Granaglione (IT); SPAGNESI, Sonia, I-59100 Prato (IT); VAGNOLI, Luana, I-AREZZO 52040 (IT); GIACOMELLI, Silvia, I-50019 Sesto Fiorentino (IT); TAMERLANI, Giancarlo, I-40030 Castel Di Casio (IT); CIPOLLETTI, Giovanni, I-20143 Milano (IT)
(74) Representative: Valenza, Silvia
(86) International application number: PCT/IB2013/055572
(87) International publication number: WO 2014/006606

(56) References cited:
- EP-A1- 0 132 998
- WO-A1-92/12263
- TOCHUKWU N. NWAGU ET AL: "Stabilization of a Raw-Starch-Digesting Amylase by Multipoint Covalent Attachment on Glutaraldehyde-Activated Amberlite Beads", JOURNAL OF MICROBIOLOGY AND BIOTECHNOLOGY, vol. 22, no. 5, 28 May 2012 (2012-05-28), pages 628-636, XP055051214, ISSN: 1017-7825, DOI: 10.4014/jmb.1108.08070
- DATABASE WPI Week 198839 Thomson Scientific, London, GB; AN 1988-273893 XP002691199, & JP 63 198985 A (SHOKUHIN SANGYO BIOREACTOR) 17 August 1988 (1988-08-17)
- AYBAST@?ER O ET AL: "Optimization of immobilization conditions of Thermomyces lanuginosus lipase on styrene@?divinylbenzene copolymer using response surface methodology", JOURNAL OF MOLECULAR CATALYSIS. B, ENZYMATIC, ELSEVIER, AMSTERDAM, NL, vol. 63, no. 3-4, 1 May 2010 (2010-05-01), pages 170-178, XP026929169, ISSN: 1381-1177, DOI: 10.1016/J.MOLCATB.2010.01.013 [retrieved on 2010-02-26]

## Description

### FIELD OF THE INVENTION

The present invention relates to the field of enzymes immobilised on polymer matrices, and in particular relates to the immobilisation of hydrolase enzymes on styrene-divinyl benzene matrices which are non-functionalised or functionalised with tertiary amine groups, by forming a stable bond between the matrix activated with glutaraldehyde and the enzyme.

### PRIOR ART

The use of immobilised enzymes on insoluble solid supports in industrial production processes has long been known. Immobilising enzyme molecules makes it possible to reuse them in subsequent reaction cycles, resulting in reduced production costs and simplified operations for isolating the products of the enzyme reaction. For this purpose, it has been found to be necessary to research particularly economical insoluble supports and simple and efficient immobilisation methods.

The most common immobilisation techniques can be divided into four categories:
1) adsorption with the formation of weak ionic or Van der Waals bonds between the support and the enzyme;
2) entrapment of the enzyme molecules in polymer gels;
3) crosslinking with the formation of covalent bonds between the different protein molecules; said cross-linking can be carried out on the enzyme itself, after adsorption on a polymer matrix free of primary amine groups or after entrapment in a polymer gel;
4) covalent bonding with formation of a stable bond between the support and the enzyme.

Adsorption is a simple and economical method, but has the drawback that the enzyme molecules can easily be desorbed from the carrier; the desorption may be caused even by small changes in the reaction conditions such as variation in the pH, salinity or concentration of the support. Even with entrapment, losses of activity due to the enzyme leaving the reticule of the gel may be found. These drawbacks can be eliminated in part or reduced by way of cross-linking between the different enzyme molecules, but this technology is not applicable to all supports and all enzymes, and often leads to considerable losses of activity in terms of the use of the free enzyme. The formation of a covalent bond with a solid insoluble matrix is an effective method for stabilising the immobilisation of the enzyme. However, not all carriers have suitable functional groups for the formation of covalent bonds, generally by way of a divalent linker which makes it possible to form Schiff bases, between the solid support and the linker and between the linker and the enzyme. Also, in many cases the cost of the carrier or the complexity of the immobilisation process makes this method economically disadvantageous in comparison to the use of the free enzyme.

The use of bifunctional binders such as glutaraldehyde for immobilising the enzymes by way of the covalent bond or cross-linking is a relatively cheap and very common method (David R. Walt, Venetka I. Agayn, The chemistry of enzyme and protein immobilization with glutaraldehyde, TrAC Trends in Analytical Chemistry, Volume 13, Issue 10, November-December 1994, pages 425-430*,* P. Monsan, Optimization of glutaraldehyde activation of a support for enzyme immobilization, Journal of Molecular Catalysis, volume 3, Issue 5, February 1978, pages 371-384*,* HeeJeongChae, Man-Jin In and Eui Yong Kim Optimization of protease immobilization by covalent binding using glutaraldehyde Applied Biochemistry and Biotechnology Volume 73, Numbers 2-3 (1998), pages 195-204).

In this case the immobilisation may basically take place in two ways:
1) if there are any primary amine groups on the insoluble support, glutaraldehyde activation of the primary amine groups and subsequent bonding of the enzyme to the activated matrix; in this case, an aldehyde group of the glutaraldehyde reacts with an amine group of the solid support, forming a Schiff base, and subsequently the other aldehyde group of the glutaraldehyde reacts with primary amine groups (generally side chains of lysine residues) on the enzyme.
2) if there are no primary amine groups on the insoluble support, and it is thus not possible to form the Schiff base between the support and an aldehyde group of the glutaraldehyde, adsorption or entrapment of the enzyme molecules onto the support and subsequent stabilisation by glutaraldehyde cross-linking; in this case, the enzyme molecules are interconnected by way of bonds established with the linker, and this cross-linking may have a negative effect on the enzyme activity or the possibility of reuse for a number of cycles (Lorena Betancor, et al, Glutaraldehyde in Protein Immobilization - A Versatile reagent - Methods in Biotechnology™, 1, Volume 22, Immobilization of Enzymes and Cells, pages 57-64, Bayraktar H,et al. Immobilization and stabilization of α-galactosidase on Sepabeads EC-EA and EC-HA., Int J BiolMacromol 2011 Nov 1;49(4):855-60*. Epub 2011 Aug 19).*

Thus for example in JP3015387 the primary amine groups of the carrier (polyglycidyl methacrylate functionalised with ethanolamine) are activated with a glutaraldehyde solution buffered to pH 8 and subsequently the support prepared in this manner is placed in contact with a solution containing the enzyme in the same buffer.

In CN101560511 the enzyme fructosyltransferase is initially adsorbed onto the macroporous support, by way of ionic bonding, and subsequently stabilised by cross-linking with a glutaraldehyde solution. The activity of the enzyme can reach 81 % and it can be recycled approximately 10 times.

The various insoluble supports, derivatised with primary amine groups, which are used for immobilisation with glutaraldehyde include acrylic matrix polymer resins (such as Sepabeads® EC-EA Resindion, Sepabeads® EC-HA Resindion, PUROLITE® ECR8310F and PUROLITE® ECR8310F), styrene-divinyl benzene matrix polymer resins (such as PUROLITE® A109) and polysaccharide matrix resins (such as EAH Sepharose™ 4B). These supports are commercially available almost exclusively for special applications (and generally on a laboratory scale), just like immobilising enzymes, and are generally the result of rather expensive production processes. As a result, these supports have very high costs, which limit the use thereof on an industrial scale.

This results in the need to have enzymes immobilised on cheaper resins available, and therefore also the need for methods for immobilising enzymes on cheaper resins by way of stable bonds. Of the cheaper resins, those which are functionalised with tertiary amines, such as PUROLITE® A100, PUROLITE® A100S, PUROLITE® A120 S, Rohm and Haas Amberlite™ IRA-94S, and Rohm and Haas Amberlite™ FPA 51, and those which are non-functionalised, such as Rohm and Haas Amberlite™ FPX66 and Rohm and Haas Amberlite™ FPX68 - which are marketed for large-scale applications, for example for producing demineralised water or for deionising low-added-value solutions - are commercially available.

Since the aforementioned resins, which are non-derivatised or carry tertiary amine groups, theoretically cannot form Schiff bases, it is assumed that they cannot be used for immobilising enzymes by forming a covalent bond with glutaraldehyde, but at the very most by adsorption and subsequent cross-linking with glutaraldehyde.

There is therefore a clear need to provide a process for immobilising enzymes, by way of stable bonds, on cheap polymer matrices which are free of primary amine groups.

There is also a need to have enzymes available, in particular hydrolase enzymes, which are immobilised on solid supports, possibly very cheap ones, by way of stable bonds so as to preserve the enzyme effectiveness in stressful reaction conditions such as high salinity or high temperature and for a high number of reaction cycles, so as to be able to use them for industrial-scale production.

### SUMMARY OF THE INVENTION

Surprisingly, it has been found that non-derivatised polymer resins based on styrene-divinyl benzene copolymer, which theoretically cannot form Schiff bases, react with bifunctional binders, in particular with glutaraldehyde, so as to form a covalent bond and lead to an activated intermediate which in turn is particularly suitable for immobilising enzymes by way of covalent bonding. The enzymes immobilised in this manner are particularly stable and maintain the catalytic efficiency thereof for a very large number of reaction cycles, even if they are kept in extreme temperature and salinity conditions for long periods.

The present disclosure relates to a polymer support having a styrene-divinyl benzene copolymer structure, which is activated by treatment with a dialdehyde compound of formula OHC-(CH₂)ₙ-CHO, where n is a whole number between 1 and 10, n is preferably between 2 and 4, more preferably n=3 which is glutaraldehyde, where said polymer support, before being activated, is non-functionalised or is functionalised with amine or ammonium groups excluding primary amine groups, wherein a covalent bond is formed between said copolymer and the dialdehyde compound (for example glutaraldehyde).

The activated support according to the present disclosure is stable and can be stored for months while maintaining the reactivity thereof in successive uses.

The present disclosure thus further relates to a method for preparing the aforementioned activated support and to the use thereof for immobilising an enzyme thereon by forming a covalent bond between the enzyme and an aldehyde group of the dialdehyde compound of formula OHC-(CH₂)ₙ-CHO (for example glutaraldehyde) bonded to the support.

The disclosure thus further relates to an enzyme immobilised on an activated support as disclosed above and to the method for preparing said immobilised enzyme.

The present disclosure combines the advantages of a simple and effective immobilisation method with the use of cheap commercial resins such as PUROLITE® A100, PUROLITE® A100S, PUROLITE® A120 S, Rohm and Haas Amberlite™ IRA-94S, and Rohm and Haas Amberlite™ FPA 51.

In this case, contrary to what is known in the art, the same procedure is used for activating the derivatised resins with primary amine groups on tertiary resins which are free of amine groups and thus (theoretically) chemically unavailable for the formation of Schiff bases with glutaraldehyde, leading, surprisingly, to comparable results in both cases. Thus, for the present invention, the nature of the insoluble matrix is particularly important, and it must necessarily be formed of a styrene-divinyl benzene polymer. In fact, the same procedure does not lead to the same result as the present invention when applied to tertiary amine resins but with a different matrix (for example polymethacrylic).

The immobilised enzymes according to the present disclosure had an increased stability of activity both in conditions of extreme salinity and in conditions of operating temperatures for continuous cycle operations for over 2 months.

The immobilised enzymes according to the present disclosure were particularly useful in the industrial production processes of galactose and tagatose.

### DETAILED DESCRIPTION OF THE INVENTION

Preferably, according to the present disclosure, the starting support for preparing the activated support is selected from the group of PUROLITE® A100, PUROLITE® A100S, PUROLITE® A120 S, Rohm and Haas Amberlite™ IRA-94S, Rohm and Haas Amberlite™ FPA 51, Rohm and Haas Amberlite™FPX66 and Rohm and Haas Amberlite™FPX68.

Preferably, according to the disclosure, a solution of dialdehyde compound (preferably glutaraldehyde) at 1-50 % by weight, more preferably at 3-7 %, buffered to a suitable pH generally of between 2 and 9, preferably 5, is used for activating said support.

The activaction treatment using the solution of dialdehyde compound is performed at a temperature of between 4 and 60 °C, preferably at room temperature (20-30 °C), for 2-120 hours, preferably 18-36 hours, while keeping the mixture stirring slowly.

The activated resin which is thus obtained is subsequently isolated by filtration and washed with water and subsequently with a solution buffered to the same pH as that used for the activation.

The activated resin may be used for immobilising enzymes by way of covalent bonding (between the aldehyde functions and an amine group of the enzyme).

For immobilising the enzyme, it is preferable to prepare a solution of the enzyme, buffered to pH 2-9 (depending on the enzyme), at a concentration of on average 50-5000 ppm of total proteins in solution, which is added to the resin activated as disclosed above. The mixture is kept stirring slowly at a temperature of between 4 and 60 °C, preferably at a temperature of 20-30 °C, for 6-240 hours. Like the pH, the immobilisation time varies between enzymes.

Once immobilisation has been achieved, the resin is subsequently isolated by filtration and washed with water and subsequently with a buffered solution.

The immobilised enzyme which is thus obtained can be used in accordance with the optimum operating conditions thereof.

For the purposes of the present disclosure, any enzyme can be immobilised, and the enzyme is preferably selected from the hydrolases and the isomerases, thus for example: beta-galactosidase, L-arabinose isomerase, α-fucosidase.

In a particularly preferred embodiment, the enzyme beta-galactosidase immobilised according to the present invention was particularly advantageously used for preparing tagatose from lactose contained in mother liquors of crystallisation or else in whey permeate.

In particular, the present invention further relates to a process for preparing tagatose from lactose, said process comprising the following steps:
a- hydrolysing the lactose with beta-galactosidase enzyme immobilised as disclosed above, to give glucose and galactose;
   wherein preferably, because it is particularly suitable, said lactose is that contained in:
   - mother liquors of crystallisation, from an industrial production process, having a conductivity of approximately 24 mS/cm;
      whey permeate with 21-23 % dry matter with approximately 18 % lactose and a conductivity of approximately 10 mS/cm.
b- deglucosation with baker's yeast;
c- epimerising the galactose to tagatose by way of aerated lime.

Optionally, the above process may further comprise the following step:
d- final degalactosation with yeast to obtain a galactose-free product.

The present invention will be better understood in the light of the following practical examples.

### EXPERIMENTAL PART

### EXAMPLE 1

### Immobilisation of the enzyme lactase (β-galactosidase) from Aspergillus orizae on tertiary amine styrene-DVB resin according to the present disclosure - laboratory scale

62.5 ml of Amberlite™Rhom and Haas FPA 51 styrene-divinyl benzene polymer matrix resin, functionalised with tertiary amine groups, were washed on a Gooch filter funnel under vacuum with 400 ml of demineralised water and subsequently with 250 ml of an aqueous solution of Na acetate 100 mM at pH 5 at room temperature. After washing, the resin was transferred into a 250 ml glass flask provided with a stirring rod, combined with 43 ml of an aqueous solution of Na acetate 100 mM at pH 5 containing 5 % glutaraldehyde, and kept stirring slowly at room temperature for 24 hours. From the HPLC analysis of the samples of the reaction supernatant, the following results were observed:
[Initial glutaraldehyde] = 5.95 %
[Final glutaraldehyde] = 3.22 %

The resin was subsequently transferred into a Gooch filter funnel and washed with 400 ml of demineralised water and with 250 ml of an aqueous solution of Na acetate 100 mM at pH 5 at room temperature.

2 g of lactase enzyme (β-galactosidase) Kerry Biolactase F CONC were dissolved in 175 ml of an aqueous solution of Na acetate 100 mM at pH 5 in a 250 ml glass flask provided with a stirring rod, into which the activated and filtrated resin was transferred, and were kept stirring slowly at room temperature for 24 hours.

The resin was recovered and washed with 40 ml of demineralised water.

From the analysis of the total proteins in the immobilisation supernatant by the Bio-Rad method, the following results were obtained:
[Total proteins prior to immobilisation] = 1650 µg/ml
[Total proteins after immobilisation] = 11 µg/ml

Therefore, the percentage of immobilised proteins on the resin turns out greater than 90 %.

The activity of the immobilised enzyme was verified by transferring 10 ml of derivatised resin into a 2.5 litre glass reactor and combining it with 2 kg of a 20 %

(W/W) aqueous solution of lactose at pH 4.9 with slow stirring at a temperature of 50 °C. During the hydrolysis reaction, samples were taken for HPLC, obtaining the following results:

| Time | Lactose % (W/W) | Glucose % (W/W) | Galactose % (W/W) |
|---|---|---|---|
| Start | 17.3 | - | - |
| 24 hours | 5.6 | 6.4 | 4.1 |
| 120 hours | 1.1 | 9.3 | .4 |

### EXAMPLE 2

### Immobilisation of the enzyme lactase (β-galactosidase) from Aspergillusorizae on non-derivatised styrene-DVB resin

62 ml of Rhom and Haas Amberlite™ FX 68non-derivatised styrene-divinyl benzene polymer matrix resin were washed on a Gooch filter funnel under vacuum with 400 ml of demineralised water and subsequently with 250 ml of an aqueous solution of Na acetate 100 mM at pH 5 at room temperature. After washing, the resin was transferred into a 250 ml glass flask provided with a stirring rod and combined with 43 ml of an aqueous solution of Na acetate 100 mM at pH 5 containing 5 % glutaraldehyde and kept stirring slowly at room temperature for 24 hours. From the HPLC analysis of the samples of the reaction supernatant, the following results were observed:
[Initial glutaraldehyde] = 4.35%
[Final glutaraldehyde] = 1.99 %

The resin was subsequently transferred into a Gooch filter funnel and washed with 400 ml of demineralised water and with 250 ml of an aqueous solution of Na acetate 100 mM at pH 5 at room temperature.2 g of lactase enzyme (β-galactosidase) Kerry Biolactase F CONC were dissolved in 175 ml of an aqueous solution of Na acetate 100 mM at pH 5 in a 250 ml glass flask provided with a stirring rod, into which the activated and filtrated resin was transferred, and were kept stirring slowly at room temperature for 24 hours.

The resin was recovered and washed with 400 ml of demineralised water.

From the analysis of the total proteins in the immobilisation supernatant by the Bio-Rad method, the following results were obtained:
[Total proteins prior to immobilisation] = 1298µg/ml
[Total proteins after immobilisation] = 69µg/ml

Therefore, the percentage of immobilised proteins on the resin turns out greater than 90 %.

The activity of the immobilised enzyme was verified by transferring 25 ml into a 1 litre glass reactor equipped with a stirring rod, temperature-controlled to 50 °C and containing 500 g of 20 % (W/W) lactose solution at pH 4.9.

During the hydrolysis reaction, samples were taken at various times for HPLC analysis, and gave the following results:

| Time | Lactose % (W/W) | Glucose % (W/W) | Galactose % (W/W) |
|---|---|---|---|
| Start | 18.0 | - | - |
| 24 hours | 0.6 | 8.9 | 8.3 |

The same resin was used for a second hydrolysis reaction, prepared as above, obtaining the following results:

| Time | Lactose % (W/W) | Glucose % (W/W) | Galactose % (W/W) |
|---|---|---|---|
| Start | 20.0 | - | - |
| 24 hours | 0.5 | 10.1 | 8.3 |

### EXAMPLE 3 - comparative example

### Immobilisation of the enzyme lactase (β-galactosidase) from Aspergillusorizae on a primary amine styrene resin

13.4 Kg of Purolite ® A 109 styrene-divinyl benzene polymer matrix resin functionalised with primary amino groups were introduced into a steel filter under vacuum and washed, at room temperature, with 2 aliquots of 25 litres each of isopropyl alcohol, with 3 aliquots each of 25 litres of demineralised water and with 25 litres of a solution of Na acetate 100 mM at pH 5. The resin was transferred into a 250 litre steel reactor, combined with 70 litres of an aqueous solution of Na acetate100 mM at pH 5 containing 5% glutaraldehyde and kept stirring slowly at room temperature for 24 hours. From the HPLC analysis of the supernatant of the reaction, the following results were observed:
[Initial glutaraldehyde] = 5.57%
[Final glutaraldehyde] = 3.35 %

The resin was subsequently transferred into a steel filter under vacuum and washed with 100 litres of demineralised water and with 25 litres of a solution of Na acetate 100 mM at pH 5.

400 g of lactase enzyme (β-galactosidase) Kerry Biolactase F CONC were dissolved in 60 litres of an aqueous solution of an Na acetate 100 mM solution at pH 5 in a 250 litre steel reactor and combined with the resin while stirring slowly at room temperature for 72 hours.

From the analysis of the total proteins in the immobilisation supernatant by the Bio-Rad method, the following results were obtained:
[Total proteins prior to immobilisation] = 1200 µg/ml
[Total proteins after immobilisation] = 10 µg/ml

Therefore, the percentage of immobilised proteins on the resin turns out greater than 90 %.

The activity of the immobilised enzyme was verified by transferring 10 ml of derivatised resin into a 2.5 litre glass reactor and combining it with 2 kg of a 20 % (W/W) aqueous solution of lactose at pH 4.8 while stirring slowly at a temperature of 50 °C. During the hydrolysis reaction, samples were taken at various times for HPLC analysis, and gave the following results:

| Time | Lactose % (W/W) | Glucose % (W/W) | Galactose % (W/W) |
|---|---|---|---|
| Start | 19.3 | - | - |
| 24 hours | 5.8 | 5.9 | 3.8 |
| 96 hours | 1.0 | 8.7 | 7.8 |

### EXAMPLE 4 - comparative example

### Immobilisation of the enzyme lactase (β-galactosidase) from Aspergillus orizae on non-styrenic amine resin

33.5 g of Rhom and Haas Amberlite™ FPA 55 acrylic matrix resin were washed on a Gooch filter funnel under vacuum with 125 ml of isopropyl alcohol, with 400 ml of demineralised water and brought to equilibrium with 250 ml of an aqueous solution of Na acetate 100 mM at pH 5. The resin was combined, in a 250 ml glass flask equipped with a stirring rod, with 175 ml of an aqueous solution of Na acetate 100 mM at pH 5 containing 5 % glutaraldehyde and kept stirring slowly at room temperature for 24 hours.

The resin was subsequently transferred into a Gooch filter funnel and washed with 400 ml of demineralised water and with 250 ml of an aqueous solution of Na acetate 100 mM at pH 5 at room temperature. 2 g of lactase enzyme (β-galactosidase) Kerry Biolactase F CONC were dissolved in 175 ml of an aqueous solution of Na acetate 100 mM at pH 5 in a 250 ml glass flask equipped with a stirring rod. The resin was combined with the enzyme solution and kept stirring slowly at room temperature for 24 hours.

The resin was recovered and washed with 400 ml of demineralised water.

From the analysis of the total proteins in the immobilisation supernatant by the Bio-Rad method, the following results were obtained:
[Total proteins prior to immobilisation] = 2108 µg/ml
[Total proteins after immobilisation] = 1632 µg/ml

Therefore, the percentage of immobilised proteins on the resin turns out greater than 22.6%.

The activity of the immobilised enzyme was verified by transferring 7.5 ml of derivatised resin into a 2.5 litre glass reactor and combining it with 1.5 kg of 20 % (W/W) aqueous lactose solution at pH 4.8 while stirring at a temperature of approximately 50 °C. During the hydrolysis reaction, samples were taken for HPLC analysis, and gave the following results:

| Time | Lactose % (W/W) | Glucose % (W/W) | Galactose % (W/W) |
|---|---|---|---|
| Start | 19.0 | - | - |
| 24 hours | 18.9 | 0.1 | 0.01 |

### EXAMPLE 5

### Immobilisation of the enzyme lactase (β-galactosidase) from Aspergillus orizae on tertiary amine styrene resin by the method of the present invention, industrial scale 1500 litres

1500 litres of Amberlite™Rhom and Haas FPA51 resin were washed, in a 10 m³ steel reactor provided with a mesh filter on the bottom valve, three times with 3 aliquots each of 1500kg of demineralised water and once with 1500kg of a solution of Na acetate 100 mM at pH 5. After washing, the solutions were drained, leaving the wet resin to deposit on the base of the reactor.

To the resin were subsequently added 950 kg of a solution of Na acetate 100 mM at pH 5 and 105 kg of a 50 % (W/W) aqueous solution of glutaraldehyde and kept stirring slowly at 25 °C for 30 hours. From the HPLC analysis of the supernatant of the reaction, the following results were observed:
[Initial glutaraldehyde] = 1.83%
[Final glutaraldehyde] = 0.51 %

After discharging the reaction supernatant, the resin was washed three times with 3 aliquots each of 1500 kg of demineralised water and once with 1500kg of a solution of Na acetate 100 mM at pH 5. To the resin were added 4000 kg of a solution ofNa acetate 100 mM at pH 5 and 60 kg of lactase enzyme (β-galactosidase) Kerry Biolactase F CONC while stirring slowly at 25 °C for 65 hours. During the reaction, samples of supernatant were taken at various times so as to determine the total proteins by the Bio Rad method, giving the following results:
[Total proteins at start of reaction] = 1053µg/ml
[Total proteins after 48 hours] = 11.1 µg/ml
[Total proteins after 61 hours] = 10.3 µg/ml
[Total proteins after 65 hours] = 11.1 µg/ml

Therefore, the percentage of immobilised proteins on the resin was greater than 90 % after 48 hours.

The resin was subsequently washed with 4000 kg of demineralised water and discharged wet from the reactor.

The activity of the immobilised enzyme was verified by transferring 20 ml of derivatised resin into a 1 litre glass reactor and adding 500 ml of enriched bovine milk serum in lactose at a concentration of 20 % (W/W) at pH 4.9 while stirring slowly for 45.5 hours. During the hydrolysis reaction, samples were taken for HPLC analysis, and gave the following results:

| Time | Lactose % (W/W) | Glucose % (W/W) | Galactose % (W/W) |
|---|---|---|---|
| Start | 17.9 | - | - |
| 4.5 hours | 2.7 | 7.2 | 5.6 |
| 45 hours | 0.2 | 9.2 | 9.0 |

### EXAMPLE 6

### Hydrolysis of lactose from crystallising mother liquors of high conductivity (with the resin of Example 5).

The immobilised enzyme prepared as in Example 5 was used for the hydrolysis reaction of the lactose contained in the mother liquors of crystallisation of the industrial production process of lactose, at high salinity.

Into a 250 ml glass flask equipped with a stirring rod were introduced 250 g of mother liquors of crystallisation to a conductivity of 23.6 mS/cm (at 17.5 ° C). The solution was brought to pH 5 and after heating it to a temperature of 50 ° C 12.5 ml of resin, derivatised as in Example 4, were added. The reaction was kept stirring slowly at 50 ° C for 24 hours. HPLC analysis of the samples taken at the start and at the end of the hydrolysis reaction gave the following results:

| Time | Lactose % (W/W) | Glucose % (W/W) | Galactose % (W/W) | Lactic Ac. % (W/W) |
|---|---|---|---|---|
| Start | 13.0 | - | 2.2 | 2.1 |
| 24 hours | 0.7 | 6.8 | 8.9 | 2.2 |

The resin was subsequently recovered and reused for a subsequent hydrolysis reaction, giving the following results:

| Time | Lactose % (W/W) | Glucose % (W/W) | Galactose % (W/W) | Lactic Ac. % (W/W) |
|---|---|---|---|---|
| Start | 13.0 | - | 2.2 | 2.1 |
| 24 hours | 0.4 | 7.7 | 9.9 | 2.5 |

### EXAMPLE 7

### Immobilisation of the enzyme lactase (β-galactosidase) from Bacillus circulans on tertiary amine styrene resin by the method of the present disclosure

134 g of Purolite® A 120 S styrene-divinyl benzene polymer matrix resin, functionalised with tertiary amine groups, were washed on a Gooch filter funnel under vacuum with 400 ml of demineralised water and subsequently with 250 ml of an aqueous solution of Na acetate 100 mM at pH 5 at room temperature. After washing, the resin was transferred into a 500 ml glass flask provided with a stirring rod and combined with 175 ml of an aqueous solution of Na acetate 100 mM at pH 5 containing 5 % glutaraldehyde and kept stirring slowly at room temperature for 24 hours. From the HPLC analysis of the samples of the reaction supernatant, the following results were observed:
[Initial glutaraldehyde] = 5.64 %
[Final glutaraldehyde] = 2.18 %

The resin was subsequently transferred into a Gooch filter funnel and washed under vacuum with 400 ml of demineralised water and with 250 ml of an aqueous solution of Na acetate 100 mM at pH 5 at room temperature. 40 g of lactase enzyme (β-galactosidase) from *Bacillus Circulans* Biolactasa NTL CONC Biocon were diluted to 700 ml with a solution of Na acetate 100 mM at pH 5 and combined with the resin in a 1000 ml glass flask provided with a stirring rod and kept stirring slowly for 72 hours at room temperature.

From the analysis of the total proteins in the immobilisation supernatant by the Bio-Rad method, the following results were obtained:
[Total proteins prior to immobilisation] = 849*g/ml
[Total proteins after immobilisation] = 15.1µg/ml

Therefore, the percentage of immobilised proteins on the resin turns out greater than 90 %.

The functionality of the resin was evaluated in a GOS (galacto-oligosaccharide) formation reaction, from enriched bovine milk serum in lactose to a concentration of 40 % (W/W) at pH 5.

Into a 1 litre glass reactor were introduced 400 ml of enriched serum and 16 ml of functionalised rein. The reaction was kept at a temperature of 50 °C while stirring slowly for 48. From the HPLC analysis of the samples taken during the reaction, the following results were observed:

| Time | Lactose (area %) | GOS (area %) | Glucose (area %) | Galactose (area %) |
|---|---|---|---|---|
| Start | 95 | - | - | - |
| 19 hours | 37.6 | 37.9 | 21.4 | 1.9 |
| 48 hours | 28.8 | 44.3 | 22.8 | 3.4 |

### EXAMPLE 8

### Immobilisation of the enzyme L-arabinose isomerase on tertiary amine styrene resin by the method of the present disclosure

2 litres of Amberlite™ Rhom and Haas FPA 51 were washed on a Gooch filter funnel under vacuum with 6 litres of demineralised water and subsequently with 2 litres of an aqueous solution of Na acetate 100 mM at pH 5 at room temperature. The resin was transferred into a 4-neck glass flask provided with a stirring rod, combined with 4 litres of an aqueous solution of Na acetate 100 mM at pH 5 containing 5 % glutaraldehyde and kept stirring slowly at room temperature for 72 hours. From the HPLC analysis of the samples of the reaction supernatant, the following results were observed:
[Initial glutaraldehyde] = 4.7 %
[Final glutaraldehyde] = 3.9 %

The resin was subsequently recovered and washed on a Gooch filter funnel under vacuum with 6 litres of demineralised water and subsequently with 2 litres of an aqueous solution of Na acetate 100 mM at pH 5 at room temperature.

250 ml of a solution containing 12700 ppm of heat-stable L-arabinose isomerase enzyme Nutrilab NV were diluted by adding 750 ml of Na phosphate buffer 100 mM at pH 7 and combined with 1 litre of activated resin in a 3 litre glass flask equipped with a stirring rod. The reaction was kept stirring slowly at room temperature for 96 hours.

From the analysis of the total proteins in the immobilisation supernatant by the Bio-Rad method, the following results were obtained:
[Total proteins prior to immobilisation] = 1349µg/ml
[Total proteins after immobilisation] = 96µg/ml

Therefore, the percentage of immobilised proteins on the resin turns out greater than 90 %.

The activity of the immobilised enzyme was evaluated in a conversion reaction from galactose to tagatose. For this purpose, 60 ml of derivatised resin were combined with 150 g of a 35 % (W/W) galactose solution at pH 7.3, containing MnCl₂1mM, in a 4-neck glass flask equipped with a stirring rod, having a pH-stat system and immersed in a glycerine bath temperature-controlled to 60 °C. Thereaction was kept stirring slowly for 161 hours. From the HPLC analyses of the samples taken during the reaction, the following results were observed:

| Time | Tagatose (area %_{TGT}/ area %_{TGT} + area %_{GLT}) | Galactose (area %_{GLT} / area %_{TGT} + area %_{GLT}) |
|---|---|---|
| Start | - | 100 |
| 19 hours | 7.8 | 92.2 |
| 40 hours | 10.7 | 89.3 |
| 65.5 hours | 13 | 87 |
| 88 hours | 14.5 | 85.5 |
| 161 hours | 18.4 | 81.6 |

### EXAMPLE 9

### Continuous use of the enzyme lactase (β-galactosidase) from Aspergillus orizae, immobilised on styrene-divinyl benzene polymer matrix resin derivatised with tertiary amine groups

75 ml of enzyme immobilised on a Purolite A 120 S styrene-divinyl benzene polymer matrix resin derivatised with tertiary amino groups, prepared as in example 1, were packed into a glass column (∅ 25 mm) and used for subsequent hydrolysis reactions of lactose in aqueous solution at both 10 % and 20 % (W/W). The substrate solution was prepared in a jacketed glass reactor equipped with a stirring rod, bringing it to a pH of 4.7 and to a temperature of 50 °C. The solution was loaded onto the column and made to flow continuously from the bottom upwards by means of a peristaltic pump positioned between the reactor and the column. The immobilised enzyme was used for various hydrolysis reactions carried out in a time period of approximately six months, at the end of which no appreciable loss of activity was observed. Indeed, the HPLC analysis of instantaneous samples taken at the output of the column after six months from the start of the reaction cycles showed a percentage of lactose hydrolysis> 90%.

### EXAMPLE 10

### Preparation of tagatose from the mother liquors of crystallisation of the industrial production process of lactose

450 g of the solution obtained as in example 6, by enzymatic hydrolysis of the mother liquors of crystallisation from the industrial production process of lactose, were introduced into a jacketed 1 litre glass reactor and brought to a temperature of 36 °C while stirring and with air insufflation. The solution was subjected to a deglucosation process by adding 0.6 g of freeze-dried baker's yeast *Saccharomyces cerevisiae.* The reaction was kept stirring slowly for 24 hours, at the end of which is a sample was taken for HPLC analysis, and gave the following results:

| Lactose % (W/W) | Glucose % (W/W) | Galactose % (W/W) |
|---|---|---|
| 0.33 | 0.04 | 8.65 |

In 415 g of the solution deglucosated in this manner, containing 35.9 g of galactose, were suspended 20 g of aerated lime Ca(OH)₂, and the epimerisation reaction was kept at a temperature of between 10 and 25 ° C for approximately 27 hours, at the end of which it was brought to pH 3.1 by adding 50 % sulphuric acid. During the reaction, samples were taken for HPLC analysis, and gave the following results:

| Time | Tagatose % (W/W) | Galactose % (W/W) |
|---|---|---|
| 3 hours | 2.8 | 3.0 |
| 5 hours | 4.3 | 2.0 |
| 27 hours | 4.4 | 1.1 |

470.8 g of solution were obtained, having a conductivity of approximately 32 mS/cm and containing 4.4 % (W/W) of tagatose (HPLC).

### EXAMPLE 11

### Preparation of tagatose from milk serum permeate

Into a four-neck 10 litre glass flask equipped with a stirring rod were introduced 8498 g of whey permeate having a lactose content of 14.92% (W/W). After correcting the pH to approximately 5 by adding 30 % NaOH, the temperature was brought to 50 °C and 100 ml of resin, on which the enzyme lactase (β-galactosidase) from *Aspergillusoryzae* Kerry Biolactase F Conchad been immobilised as in example 1, were introduced into the reactor. The hydrolysis reaction of the lactose was kept stirring slowly for approximately 72 hours, at the end of which a sample was taken for HPLC analysis, which gave the following results:

| Lactose % (W/W) | Glucose % (W/W) | Galactose % (W/W) |
|---|---|---|
| 0.51 | 6.7 | 6.57 |

The hydrolysed solution was recovered by simple filtration.

1001.7 g of hydrolysed whey permeate were transferred into a 2 litre jacketed glass reactor provided with mechanical stirring. By adding a 30% NaOH solution, the pH was brought to 7.05, and the temperature was adjusted to 32 °C. 5.01 g of fresh baker's yeast *Saccharomyces cerevisiae* were introduced into the reactor, and the reaction was kept stirring slowly with insufflation of air for approximately 24 hours, after which it was verified that the glucose content was less than 0.1 % (g/dl) by using colorimetric test strips Diabur-Test® Roche. After lowering the temperature to 10 °C, approximately 90 g of an aqueous suspension of 50 % aerated lime Ca(OH)₂ were introduced into the reactor. During the epimerisation reactions, samples were taken at various times for HPLC analysis, and gave the following results:

| Time | Tagatose % (W/W) | Galactose % (W/W) | Galactose % / Galactose% +Tagatose % |
|---|---|---|---|
| 2 hours | 3.68 | 1.06 | 0.22 |
| 3 hours | 3.92 | 0.83 | 0.17 |
| 4 hours | 3.89 | 0.63 | 0.13 |
| 5 hours | 4.01 | 0.55 | 0.12 |

After approximately 17 hours, the reaction was neutralised, bringing it to pH 6.3, by adding 247.4 g of 20 % sulphuric acid, and a sample was taken for HPLC analysis and gave the following results: tagatose 3.1% (W/W), galactose 0.4 % (W/W).

### Optional final degalactosation

The reactor temperature was brought to 32 °C and the pH was corrected to 7.2 by adding technical ammonia. 1.1 g of fresh baker's yeast *Saccharomyces cerevisiae* were introduced into the reactor and the degalactosation reaction was kept stirring with insufflation of air for 24 hours. HPLC analysis of the initial sample and of that taken at the end of the reaction showed that the ratio galactose % / galactose% + tagatose %had changed from 0.11 to 0.03. After removing the cells by filtration, 1283 g of a solution containing 38.2 g of tagatose (HPLC) were obtained. After deionisation on ion exchange resins, the solution was concentrated and the tagatose was crystallised directly from water.

## Claims

1. Process for preparing tagatose from lactose, said method comprising the use of an immobilised beta-galactosidase enzyme, the process **characterised in that**:
- said enzyme is immobilised on an activated polymeric support, said support having a styrene-divinyl benzene copolymer structure, which is activated by treatment with a di-aldehyde compound of formula OHC-(CH₂)ₙ-CHO, where n is a whole number between 1 and 10, where said polymer support, at the starting, is non-functionalised or is functionalised with amine or ammonium groups excluding primary amine groups, wherein a covalent bond is formed between said copolymer and the di-aldehyde compound and a covalent bond is formed between said enzyme and the di-aldehyde compound;
said lactose being that contained in:
- mother liquors of crystallisation, from an industrial production process; or
- whey permeate.

2. Process according to claim 1, comprising the following steps:
a- hydrolysing the lactose with beta-galactosidase enzyme immobilised according to claim 1, to give glucose and galactose;
wherein said lactose is preferably that contained in:
- mother liquors of crystallisation, from an industrial production process, having a conductivity of approximately 24 mS/cm;
whey permeate with 21-23 % dry matter with approximately 18 % lactose and a conductivity of approximately 10 mS/cm.
b- deglucosation with baker's yeast;
c- epimerising the galactose to tagatose by way of aerated lime.

3. Process according to claim 2, further comprising the following step:
d- final degalactosation with yeast to obtain a galactose-free product.

4. Process according to anyone of claims 1-3, wherein n is between 2 and 4.

5. Process according to anyone of claims 1-4, wherein the di-aldehyde compound is glutaraldehyde.

6. Process according to anyone of claims 1-5, wherein the starting polymer support is treated with a 1-50% by weight di-aldehyde compound solution, buffered to a suitable pH.

7. Process according to claim 6, wherein the treatment with the di-aldehyde compound solution is carried out at 4-60 °C for 2-120 hours while keeping the mixture stirring slowly.

8. Process according to any one of claims 1-7, wherein the enzyme is immobilised by adding a solution of the enzyme to the activated support.

9. Process according to claim 8, wherein the solution of the enzyme buffered to pH 2-9 is added to the activated support and the mixture is kept stirring slowly at a temperature of between 4 and 60 °C for 6-240 hours.

## Patentansprüche

1. Verfahren zur Herstellung von Tagatose aus Laktose, wobei das Verfahren die Verwendung eines immobilisierten Beta-Galaktosidase-Enzyms aufweist und **dadurch gekennzeichnet ist, dass**:
- das Enzym auf einem aktivierten Polymerträger immobilisiert wird und der Träger eine Styrol-Divinyl-Benzol-Copolymer-Struktur aufweist, die durch Behandlung mit einer Di-Aldehyd-Mischung der Formel OHC-(CH₂)ₙ-CHO aktiviert wird, wobei n eine ganze Zahl zwischen 1 und 10 ist und der Polymerträger zu Beginn oder durch Amin-Ammoniumgruppen mit Ausnahme primärer Amingruppen funktionalisiert ist, wobei eine kovalente Bindung zwischen dem Copolymer und der Di-Aldehyd-Mischung und eine kovalente Bindung zwischen dem Enzym und der Di-Aldehyd-Mischung ausgebildet wird;
wobei die Laktose diejenige ist, die in:
- Mutterlaugen von industriellen Kristallisations-Produktionsprozessen; oder
- Molkepermeat
enthalten ist.

2. Verfahren gemäß Anspruch 1, aufweisend die folgenden Schritte:
a) Hydrolysieren der Laktose mit einem gemäß Anspruch 1 immobilisierten Beta-Galaktosidase-Enzym, um Glukose und Galaktose zu geben;
wobei die Laktose vorzugsweise diejenige ist, die in:
- Mutterlaugen von industriellen Kristallisations-Produktionsprozessen aufweisend eine Leitfähigkeit von etwa 24 mS/cm;
Molkepermeat mit 21 - 23 % Trockenmasse mit etwa 18 % Laktose und einer Leitfähigkeit von etwa 10 mS/cm.
b) Deglucosierung mit Bäckerhefe;
c) Epimerisierung der Galaktose zu Tagatose durch kohlensauren Kalk.

3. Verfahren gemäß Anspruch 2, des Weiteren aufweisend den folgenden Schritt:
d) finale Degalaktosierung mit Hefe, um ein galaktosefreies Produkt zu erhalten.

4. Verfahren gemäß einem der Ansprüche 1 bis 3, wobei n zwischen 2 und 4 liegt.

5. Verfahren gemäß einem der Ansprüche 1 bis 4, wobei die Di-Aldehyd-Mischung Glutaraldehyd ist.

6. Verfahren gemäß einem der Ansprüche 1 bis 5, wobei der Ausgangs - Polymerträger mit einer 1 - 50 Gew.-%-igen Di-Aldehyd-Mischungs-Lösung behandelt wird, die auf einen geeigneten pH-Wert gepuffert ist.

7. Verfahren gemäß Anspruch 6, wobei die Behandlung mit Di-Aldehyd-Mischungs-Lösung unter langsamem Rühren der Mischung bei 4 - 60 °C für 2 - 120 Stunden durchgeführt wird.

8. Verfahren gemäß einem der Ansprüche 1 bis 7, wobei das Enzym durch Zugabe einer Lösung des Enzyms zu dem aktivierten Träger immobilisiert wird.

9. Verfahren gemäß Anspruch 8, wobei die Lösung des auf einen pH-Wert von 2 - 9 gepufferten Enzyms, dem aktivierten Träger zugegeben wird und die Mischung bei einer Temperatur von 4 - 60 °C für 6 - 240 Stunden langsam gerührt wird.

## Revendications

1. Procédé de préparation de tagatose à partir de lactose, ledit procédé comprenant l'utilisation d'une enzyme de bêta-galactosidase immobilisée, le procédé étant caractérisé en ce qui :
- ladite enzyme est immobilisée sur un support polymérique activé, ledit support ayant une structure de copolymère styrène-divinyl benzène, qui est activée par traitement avec un composé di-aldéhyde de formule OHC-(CH₂)ₙ-CHO, où n est un nombre entier entre 1 et 10, où ledit support polymère, au départ, est non fonctionnalisé ou est fonctionnalisé avec des groupes amines ou ammonium à l'exclusion de groupes amines primaires, dans lequel une liaison covalente est formée entre ledit copolymère et le composé di-aldéhyde et une liaison covalente est formée entre ladite enzyme et le composé di-aldéhyde ;
ledit lactose étant celui contenu dans :
- des liqueurs mères de cristallisation, issues d'un procédé de production industrielle ; ou
- un perméat de lactosérum.

2. Procédé selon la revendication 1, comprenant les étapes suivantes :
a- l'hydrolyse du lactose avec une enzyme de bêta-galactosidase immobilisée selon la revendication 1, pour donner du glucose et du galactose ;
dans lequel ledit lactose est de préférence celui contenu dans :
- des liqueurs mères de cristallisation, issues d'un procédé de production industrielle, ayant une conductivité d'approximativement 24 mS/cm ;
un perméat de lactosérum avec 21 à 23 % de matière sèche avec approximativement 18 % de lactose et une conductivité d'approximativement 10 mS/cm.
b- la déglucosation avec de la levure de boulanger ;
c- l'épimérisation du galactose en tagatose par l'intermédiaire de chaux aérienne.

3. Procédé selon la revendication 2, comprenant en outre l'étape suivants :
d- la dégalactosation finale avec de la levure pour obtenir un produit exempt de galactose.

4. Procédé selon l'une quelconque des revendications 1 à 3, dans lequel n est entre 2 et 4.

5. Procédé selon l'une quelconque des revendications 1 à 4, dans lequel le composé di-aldéhyde est le glutaraldéhyde.

6. Procédé selon l'une quelconque des revendications 1 à 5, dans lequel le support polymère de départ est traité avec une solution de composé di-aldéhyde de 1 à 50 % en poids, tamponnée à un pH approprié.

7. Procédé selon la revendication 6, dans lequel le traitement avec la solution de composé di-aldéhyde est réalisé à 4 à 60 °C pendant 2 à 120 heures tout en conservant le mélange sous agitation lente.

8. Procédé selon l'une quelconque des revendications 1 à 7, dans lequel l'enzyme est immobilisée en ajoutant une solution de l'enzyme au support activé.

9. Procédé selon la revendication 8, dans lequel la solution de l'enzyme tamponnée à un pH de 2 à 9 est ajoutée au support activé et le mélange est conservé sous agitation lente à une température entre 4 et 60 °C pendant 6 à 240 heures.
